(19) Europäisches Patentamt
European Patent Office
Office européen des brevets

(11) Veröffentlichungsnummer: **0 552 679 A1**

# (12) EUROPÄISCHE PATENTANMELDUNG

(21) Anmeldenummer: **93100564.9**

(22) Anmeldetag: **15.01.93**

(51) Int. Cl.5: **C07D 493/04**, C07B 53/00, C07F 13/00, //(C07D493/04, 311:00,303:00)

(30) Priorität: **18.01.92 DE 4201192**

(43) Veröffentlichungstag der Anmeldung: **28.07.93 Patentblatt 93/30**

(84) Benannte Vertragsstaaten: **AT BE CH DE DK ES FR GB GR IE IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Englert, Heinrich, Dr.**
**Stormstrasse 13**
**W-6238 Hofheim/Ts.(DE)**
Erfinder: **Mania, Dieter, Dr.**
**Goethestrasse 43**
**W-6240 Königstein/Ts.(DE)**
Erfinder: **Seuring, Bernhard, Dr.**
**Frankfurter Strasse 19**
**W-6238 Hofheim/Ts.(DE)**

## (54) Verfahren zur enantioselekfiven Epoxidation von in 6-Stellung substituierten Chromenen.

(57) Beschrieben wird ein Verfahren zur enantioselektiven Epoxidation von in 6-Stellung substituierten Chromenen II

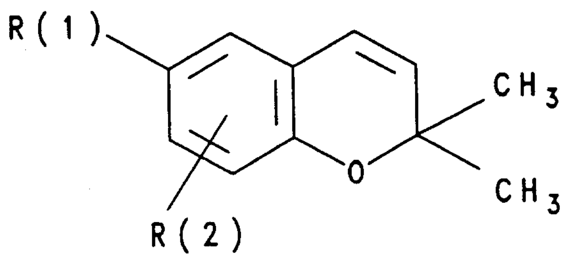

(II)

mit einem Salen-(Mangan)-Komplex der Formel III

EP 0 552 679 A1

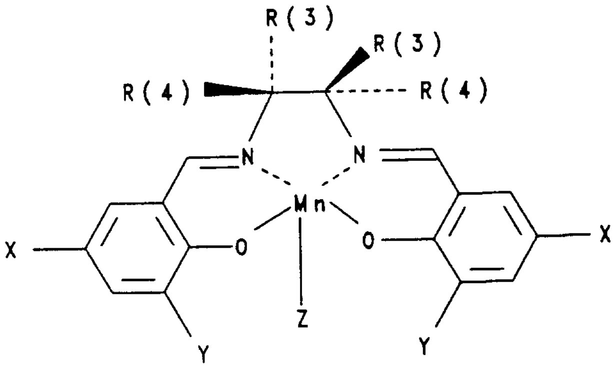

(III)

und einem Oxidationsmittel, wobei die optisch aktiven Verbindungen I erhalten werden:

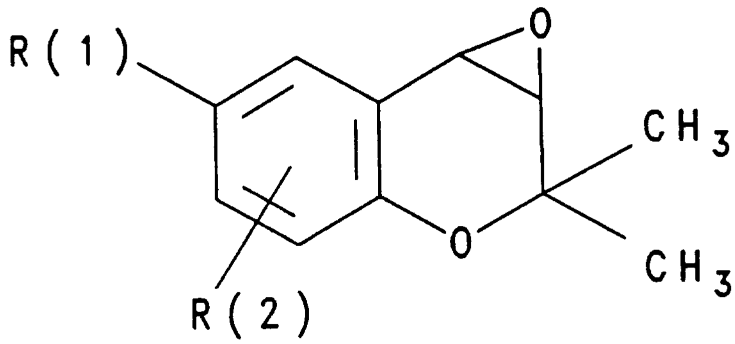

Die Erfindung betrifft ein Verfahren zum Herstellen von in 6-Stellung substituierten optisch aktiven 3,4-Dihydro-3,4-epoxy-2H-1-benzopyranen der Formel I

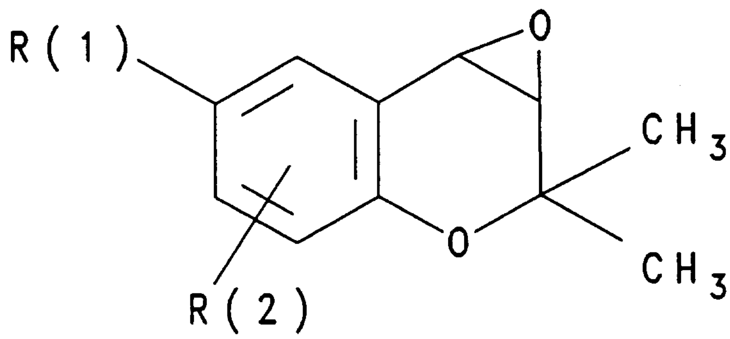

worin bedeuten:

R(1) $(C_1-C_4)$-Alkyl, $(C_1-C_4)$-Alkoxy, Di-$(C_1-C_4)$-Alkylamino, $(C_1-C_4)$-Alkyl-SO, $(C_1-C_4)$-Alkyl-$SO_2$, $CF_3$, $C_2F_5$, Cl, Br, $NO_2$, $(C_1-C_2)$-Fluoralkoxy, Ar, ArCO, ArSO, $ArSO_2$, worin Ar ein aromatisches oder heteroaromatisches System ist, welches unsubstituiert oder substituiert ist mit 1 bis 2 gleichen oder verschiedenen Resten $(C_1-C_2)$-Alkyl, $(C_1-C_2)$-Alkoxy, F, Cl, Br, $CF_3$, CN, $NO_2$, CO-$(C_1-C_2)$-Alkyl, $SO_p$-$(C_1-C_2)$-Alkyl mit p für eins oder zwei;
und Ar bedeutet als aromatisches System Phenyl, Naphthyl oder Biphenyl und als heteroaromatisches System einen fünf- oder sechsgliedrigen O-, N- und/oder S-heterocyclischen Ring, insbesondere einen Furyl-, Thienyl-, Isothiazolyl-, Oxazolyl-, Isoxazolyl-, Pyrazolyl-, Imidazolyl-, Thiazolyl-, Pyridyl-, Pyrazinyl-, Pyrimidinyl-, Pyridazinyl- oder Triazinyl-Ring,

R(2) Wasserstoff oder F, Cl, $NO_2$.

Die Erfindung umfaßt sowohl die Herstellung links- als auch rechts-drehender Verbindungen.

Bevorzugt wird das Verfahren angewendet zur Herstellung von Verbindungen der Formel I mit R(1) gleich $(C_1-C_3)$-Alkyl, Cl, Nitro, Phenylsulfinyl, Phenylsulfonyl, Benzoyl oder Phenyl, wobei die Phenylgruppe in allen Fällen in der oben angegebenen Weise substituiert sein kann, und R(2) gleich Wasserstoff.

Besonders bevorzugt hergestellte Verbindungen sind solche, in denen R(1) einen Phenylsulfinyl-, Phenylsulfonyl-, Benzoyl-Substituenten darstellt, in denen der Phenylrest in allen Fällen wie oben angegeben substituiert sein kann.

Das Verfahren ist dadurch gekennzeichnet, daß man
Chromene der Formel II

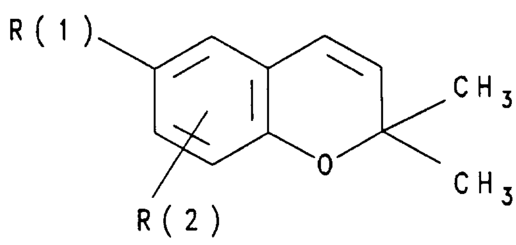

(II)

in der R(1) und R(2) die oben angegebene Bedeutung haben,
mit einem Salen-(Mangan)-Komplex der Formel III

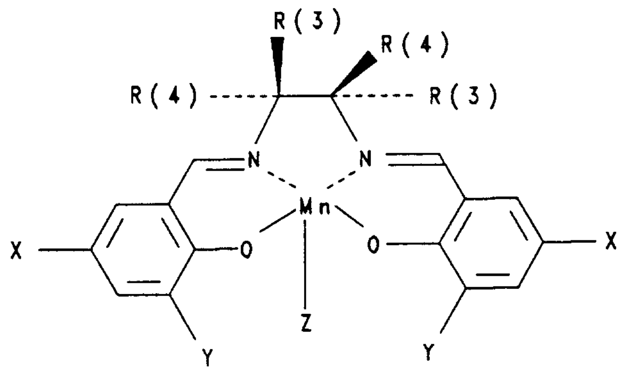

(III)

mit

R(3) und R(4) gleich H, Phenyl, Thienyl, oder

beide R(3) bilden eine $(CH_2)_n$-Kette mit n = 3 - 6 und R(4) = H, $(C_1-C_3)$-Alkyl, oder beide R(4) bilden eine $(CH_2)_n$-Kette mit n = 3 - 6 und R(3) = H, $(C_1-C_3)$-Alkyl, jedoch unter der Bedingung, daß R(3) und R(4) nicht gleich sind, und daß einer dieser beiden Substituenten Wasserstoff bedeutet,

X H, $(C_1-C_4)$-Alkyl, $(C_1-C_4)$-Alkoxy, Di-$(C_1-C_4)$-Alkylamino, $(C_1-C_4)$-Alkyl-SO, $(C_1-C_4)$-Alkyl-$SO_2$, $CF_3$, Cl, Br, $NO_2$, Adamantyl, Methylcyclohexyl

Y Di-$(C_1-C_4)$-Alkylamino, $(C_1-C_2)$-Alkyl-O, $(C_1-C_5)$-Alkyl, $(C_1-C_4)$-Alkylphenyl,

Z F, Cl, Br, Acetat, $PF_6$

und einem Oxidationsmittel umsetzt.

Alkyl bedeutet dabei stets sowohl geradkettiges als auch verzweigtes Alkyl. Als Oxidationsmittel seien substituiertes und unsubstituiertes Jodosobenzol und Chlorbleichlauge genannt.

Die Oxidation wird in einem geeigneten Lösungsmittel, vorzugsweise in einem polaren aprotischen Lösungsmittel wie Methylenchlorid, Chloroform, Acetonitril bei -10 bis 50 °C, vorzugsweise bei 0 bis 25 °C, vorgenommen, wobei der Zusatz von Donorliganden, beispielsweise Pyridin-N-oxid, 4-Phenylpyridin-N-oxid, Lutidin-N-oxid, 2-Methylimidazol, die Enantioselektivität beträchtlich erhöhen kann.

Die Aufarbeitung erfolgt durch Eintragen in Wasser, wenn Acetonitril das Lösungsmittel ist, oder besser durch Eindampfen des Lösungsmittels im Vakuum und Chromatographie des Rückstandes über eine Kieselgelsäule mit einem Gemisch aus polaren und unpolaren Lösungsmittel beispielsweise Essigester und Petrolether, Hexan, Cyclohexan.

Es ist bekannt, daß man Chromene der Formel IV

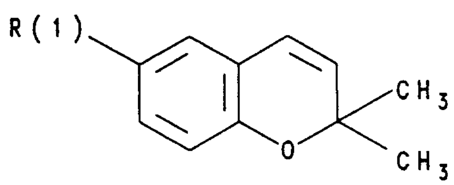

R(1) = H, CN (IV)

wie in

J. Am. Chem. Soc. 1991, 113, 6703 - 6704 sowie

J. Am. Chem. Soc. 1991, 113, 7063 - 7064 beschrieben,

zu enantioselektiven 3,4-Epoxychromanen umsetzen kann.

Die Erfindung zeigt nun, daß das Verfahren auch auf Chromene der Formel II anwendbar ist.

Die erfindungsgemäß hergestellten optisch aktiven Epoxide der Formel I sind bedeutsame Vorprodukte für die Herstellung von Verbindungen mit kaliumkanalöffnenden Eigenschaften, wie sie beispielsweise aus der US-Patentschrift 4 999 371 bekannt sind.

Beispiele

Verwendete Katalysatoren:

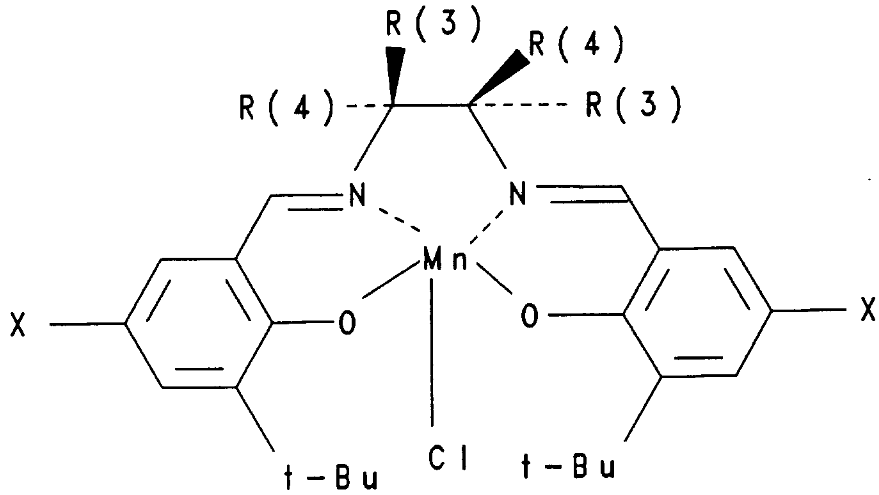

Konfiguration
(S,S)-1: R(3) = Ph, R(4) = H, X = $CH_3$
(S,S)-2: R(3) = Ph, R(4) = H, X = $OCH_3$
(R,R)-1: R(3) = H, R(4) = Ph, X = $CH_3$
(R,R)-2: R(3) = H, R(4) = Ph, X = Cl

A) Epoxidation von 2,2-Dimethyl-6-phenylsulfonyl-2H-chromen katalysiert mit (S,S)-1.

a) ohne Donor:

2,2-Dimethyl-6-phenylsulfonyl-2H-chromen (1,5 g, 5 mMol) und Jodosobenzol (1,4 g, 6,4 mMol) wurden zu einer Lösung von S,S-1 (0,1 g, 0,15 mMol) in 15 ml Acetonitril bei 20 °C eingetragen. Nach zweistündigem Rühren bei 20 °C wurde filtriert, das Filtrat im Vakuum eingedampft und der Rückstand durch Säulenchromatographie (Kieselgel, Petrolether - Essigester 3:2) gereinigt. Die vereinigten Fraktionen wurden eingedampft und der Rückstand mit ein paar Tropfen Essigester angelöst. 3,4-Dihydro-3,4-epoxy-6-phenylsulfonyl-2,2-dimethyl-2H-1-benzopyran kristallisierte nach der Zugabe von überschüssigem Diisopropylether.
Fraktion 1: 550 mg (34,8 % Ausbeute) $[\alpha]_D^{20}$ + 3,4 ° (c 1, Ethanol).
Beim Anreiben des Rückstandes der Mutterlauge mit Diisopropylether kristallisierte
Fraktion 2: 690 mg (43,6 % Ausbeute), $[\alpha]_D^{20}$ + 42,8 ° (c 1, Ethanol).

b) in Gegenwart eines Donors:

Analog Aa) mit Zusatz von 60,8 mg (0,64 mMol) Pyridin-N-oxid. Die Aufarbeitung wie unter a) beschrieben, ergab:
Fraktion 1: 460 mg (29,1 % Ausbeute), $[\alpha]_D^{20}$ + 3,4 ° (c 1, Ethanol).
Fraktion 2: 560 mg (35,4 % Ausbeute), $[\alpha]_D^{20}$ + 43,6 ° (c 1, Ethanol).

Mittels $^1$H-NMR bestimmte man für die Fraktion 2 einen Enantiomerenüberschuß von 92 % ee in Gegenwart des chiralen Shiftreagens $Eu(hfc)_3$.

B) Epoxidation von 2,2-Dimethyl-6-phenylsulfonyl-2H-chromen katalysiert mit (S,S)-2

a) ohne Donor:

Analog Aa) mit 0,1 g (0,15 mMol) Katalysator (S,S)-2, Reaktionszeit 24 Stunden.
Fraktion 1: 760 mg Ausgangsmaterial.

Fraktion 2: 350 mg 3,4-Dihydro-3,4-epoxy-6-phenylsulfonyl-2,2-dimethyl-2H-1-benzopyran $[\alpha]_D^{20}$ + 4,3° (c 1, Ethanol).

C) Epoxidation von 2,2-Dimethyl-6-phenylsulfonyl-2H-chromen katalysiert mit (R,R-1)

a) ohne Donor:

Analog Aa) mit 0,1 g (0,15 mMol) Katalysator (R,R)-1, Reaktionszeit: 2 Stunden.
Fraktion 1: 480 mg (30,4 % Ausbeute), $[\alpha]_D^{20}$ - 5,7° (c 1, Ethanol)
Fraktion 2: 590 mg (37,3 % Ausbeute), $[\alpha]_D^{20}$ - 43,2° (c 1, Ethanol)

D) Epoxidation von 2,2-Dimethyl-6-methylsulfonyl-2H-chromen katalysiert mit (S,S)-1

a) ohne Donor:

Analog Aa) mit 1,19 g (5 mMol) 2,2-Dimethyl-6-methylsulfonyl-2H-chromen und 0,1 g (0,15 mMol) Katalysator (S,S)-1,Reaktionszeit: 1 Stunde
Fraktion 1: 710 mg (55,8 % Ausbeute), $[\alpha]_D^{20}$ + 34,2° (c 1, Ethanol).
Fraktion 2: 310 mg (24,4 % Ausbeute), $[\alpha]_D^{20}$ + 55,6° (c 1, Ethanol).
Mittels $^1$H-NMR bestimmte man für die Fraktion 2 einen Enantiomerenüberschuß von 96,6 % ee in Gegenwart des chiralen Shiftreagens $Eu(hfc)_3$.

E) Epoxidation von 2,2-Dimethyl-6-methylsulfonyl-2H-chromen katalysiert mit (R,R)-2
mit Donor

Analog Aa mit 1,19 g (5 mMol) 2,2-Dimethyl-6-methylsulfonyl-2H-chromen; 0,13 g (0,2 mMol) Katalysator (R,R)-2 und 0,1 g (1 mMol) Pyridin-N-oxid, Reaktionszeit: 2 Stunden.
Der Ansatz wurde mit Aktivkohle durch ein Klärschichtfilter gesaugt, zur Trockne eingeengt und aus Diisopropylether umkristallisiert.
Ausbeute: 0,410 g (32,3 % Ausbeute), $[\alpha]_D^{20}$ -52° (c1, Ethanol)

**Patentansprüche**

**1.** Verfahren zum Herstellen von in 6-Stellung substituierten optisch aktiven 3,4-Dihydro-3,4-epoxy-2H-1-benzopyranen der Formel I

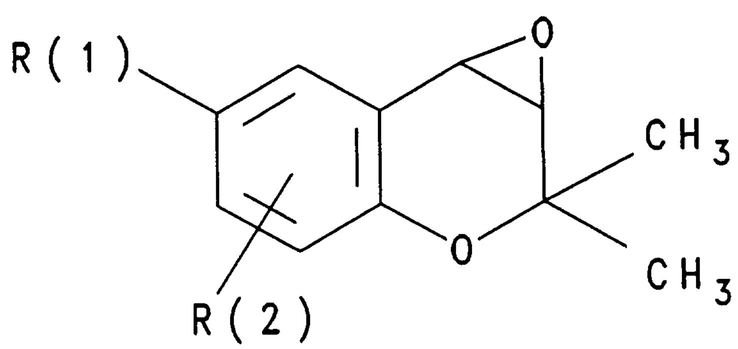

worin bedeuten:

R(1) $(C_1-C_4)$-Alkyl, $(C_1-C_4)$-Alkoxy, Di-$(C_1-C_4)$-Alkylamino, $(C_1-C_4)$-Alkyl-SO, $(C_1-C_4)$-Alkyl-$SO_2$, $CF_3$, $C_2F_5$, Cl, Br, $NO_2$, $(C_1-C_2)$-Fluoralkoxy, Ar, ArCO, ArSO, $ArSO_2$, worin Ar ein aromatisches oder heteroaromatisches System ist, welches unsubstituiert oder substituiert ist mit 1 bis 2 gleichen oder verschiedenen Resten $(C_1-C_2)$-Alkyl, $(C_1-C_2)$-Alkoxy, F, Cl, Br, $CF_3$, CN, $NO_2$, CO-$(C_1-C_2)$-Alkyl, $SO_p$-$(C_1-C_2)$-Alkyl mit p für eins oder zwei, und Ar bedeutet als aromatisches System Phenyl, Naphthyl oder Biphenyl und als heteroaromatisches System einen fünf- oder sechsgliedrigen O-, N- und/oder S-heterocyclischen Ring, insbesondere

einen Furyl-, Thienyl-, Isothiazolyl-, Oxazolyl-, Isoxazolyl-, Pyrazolyl-, Imidazolyl-, Thiazolyl-, Pyridyl-, Pyrazinyl-, Pyrimidinyl-, Pyridazinyl- oder Triazinyl-Ring,

R(2) Wasserstoff oder F, Cl, $NO_2$,

dadurch gekennzeichnet, daß man Chromene der Formel II

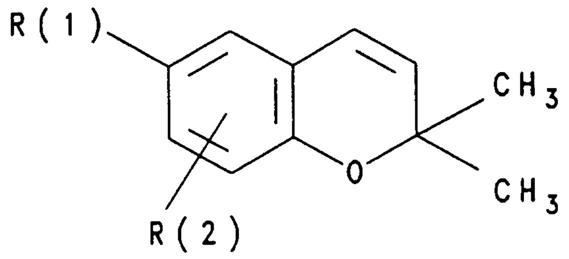

(II)

in der R(1) und R(2) die oben angegebene Bedeutung haben, mit einem Salen-(Mangan)-Komplex der Formel III

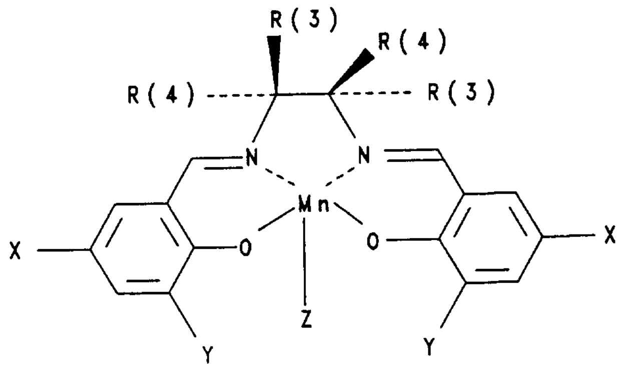

(III)

mit

R(3) und R(4) gleich H, Phenyl, Thienyl, oder

beide R(3) bilden eine $(CH_2)_n$-Kette mit n = 3 - 6 und R(4) = H, $(C_1-C_3)$-Alkyl, oder

beide R(4) bilden eine $(CH_2)_n$-Kette mit n = 3 - 6 und R(3) = H, $(C_1-C_3)$-Alkyl, jedoch unter der Bedingung, daß R(3) und R(4) nicht gleich sind und daß einer dieser beiden Substituenten Wasserstoff bedeutet,

X H, $(C_1-C_4)$-Alkyl, $(C_1-C_4)$-Alkoxy, Di-$(C_1-C_4)$-Alkylamino, $(C_1-C_4)$-Alkyl-SO, $(C_1-C_4)$-Alkyl $SO_2$, $CF_3$, Cl, Br, $NO_2$, Adamantyl, Methylcyclohexyl

Y Di-$(C_1-C_4)$-Alkylamino, $(C_1-C_2)$-Alkyl-O, $(C_1-C_5)$-Alkyl, $(C_1-C_4)$-Alkylphenyl,

Z F, Cl, Br, Acetat, $PF_6$

und einem Oxidationsmittel umsetzt.

**2.** Verfahren nach Anspruch 1, dadurch gekennzeichnet, daß man Verbindungen der Formel I mit R(1) gleich $(C_1-C_3)$-Alkyl, Cl, Nitro, Phenylsulfinyl, Phenylsulfonyl, Benzoyl oder Phenyl herstellt, wobei die Phenylgruppe in allen Fällen unsubstituiert oder substituiert ist mit 1 bis 2 gleichen oder verschiedenen Resten $(C_1-C_2)$-Alkyl, $(C_1-C_2)$-Alkoxy, F, Cl, Br, $CF_3$, CN, $NO_2$, CO-$(C_1-C_2)$-Alkyl, $SO_p$-$(C_1-C_2)$-Alkyl mit p für eins oder zwei, und R(2) gleich Wasserstoff.

**3.** Verfahren nach Anspruch 1, dadurch gekennzeichnet, daß man Verbindungen der Formel I mit R(1) gleich Phenylsulfinyl, Phenylsulfonyl, Benzoyl herstellt, wobei die Phenylgruppe in allen Fällen unsubstituiert oder substituiert ist mit 1 bis 2 gleichen oder verschiedenen Resten $(C_1-C_2)$-Alkyl, $(C_1-C_2)$-Alkoxy, F, Cl, Br, $CF_3$, CN, $NO_2$, CO-$(C_1-C_2)$-Alkyl, $SO_p$-$(C_1-C_2)$-Alkyl mit p für eins oder zwei.

Europäisches Patentamt

# EUROPÄISCHER RECHERCHENBERICHT

Nummer der Anmeldung

EP 93 10 0564 .9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | WO-A-9 114 694 (RESEARCH CORPORATION TECHNOLOGIES)<br>* Anspruch 66 *<br>--- | 1-3 | C07D493/04<br>C07B53/00<br>C07F13/00<br>//(C07D493/04, 311:00,303:00) |
| X | TETRAHEDRON LETTERS.<br>Bd. 32, Nr. 38, 16. September 1991, OXFORD GB<br>Seiten 5055 - 5058<br>NAM HO LEE ET AL 'Enantiomerically pure epoxychromans via asymmetric catalysis'<br>* Tabelle 1, Verbindungen 7,8 *<br>--- | 1-3 | |
| A | EP-A-0 337 179 (HOECHST AKTIENGESELLSCHAFT)<br>* Seite 9, Zeile 1-3 *<br>----- | 1-3 | |
| | | | RECHERCHIERTE SACHGEBIETE (Int. Cl.5 ) |
| | | | C07D<br>C07B<br>C07F |

Der vorliegende Recherchenbericht wurde für alle Patentansprüche erstellt

| Recherchenort | Abschlußdatum der Recherche | Prüfer |
|---|---|---|
| DEN HAAG | 07 JUNI 1993 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument